# EUROPÄISCHE PATENTSCHRIFT

(11) **EP 2 316 351 B1**
(45) Veröffentlichungstag und Bekanntmachung des Hinweises auf die Patenterteilung: **20.04.2016**
(21) Anmeldenummer: 10188207.4
(22) Anmeldetag: 20.10.2010
(51) Int. Cl.: A61B 17/10, A61B 17/00, A61B 1/31, A61B 17/122, A61B 17/128, A61B 17/32, A61B 17/29

(54) **Medizinisches Instrument zum Setzen von Gewebeklammern**
Medical instrument for setting tissue clamps
Instrument médical destiné à installer des pinces pour tissu

(30) Priorität: 30.10.2009 DE 102009051408
(43) Veröffentlichungstag der Anmeldung: 04.05.2011
(73) Patentinhaber: Ovesco Endoscopy AG, 72074 Tübingen (DE)
(72) Erfinder: Ho, Chi-Nghia, 70180 Stuttgart (DE); Anhöck, Gunnar, 72762 Reutlingen (DE); Baur, Franziska, 73265 Dettingen unter Teck (DE); Schurr, Marc O., 72072 Tübingen (DE); Proßt, Rüdiger, 70180 Stuttgart (DE); Gottwald, Thomas, 82431 Kochel am See (DE)
(74) Vertreter: Winter, Brandl, Fürniss, Hübner, Röss, Kaiser, Polte - Partnerschaft mbB

(56) Entgegenhaltungen:
- EP-A2- 0 634 146
- WO-A1-99/25255
- DE-U1-202008 007 774
- US-A1- 2002 062 130
- US-A1- 2003 028 207
- US-A1- 2005 177 168
- US-A1- 2005 283 189
- US-A1- 2008 249 561

## Beschreibung

Die vorliegende Erfindung betrifft ein medizinisches, vorzugsweise proktologisches Instrument zum Setzen von Gewebeklammern gemäß dem Oberbegriff des Patentanspruchs 1.

Eine derartige Vorrichtung verwendet ein schaftartiges Zuführmittel für einen Gewebeclip zum Verschließen einer Gewebeverletzung wie beispielsweise eine Analfistel und dergleichen krankhafte Gewebeveränderungen.

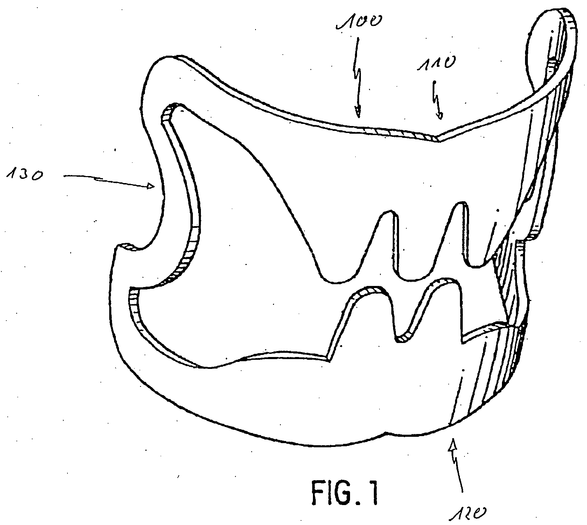

Aus dem Stand der Technik beispielsweise gemäß der US 6,849,078 B2 ist ein Gewebeclip dieser Gattung hinsichtlich seiner grundsätzlichen Konstruktion allgemein bekannt. Zum besseren Verständnis wird dieser Clip nachstehend unter Verweis auf die Fig. 1 näher beschrieben.

Demzufolge besteht ein solcher Clip 100 aus einer maulartigen Klemmeinrichtung mit zwei gezahnten Kiefern 110, 120, welche über zwei seitliche Scharniere 130 bzw. über flexible Ausformungen auf- und zugeklappt werden können. Die Scharniere 130 bzw. die flexiblen Ausformungen sind dabei vorzugsweise aus federelastischen Bändern ausgebildet, welche beim Aufklappen der Kiefer 110, 120 eine Federenergie speichern, die beim Freigeben der Kiefer 110, 120, d.h. bei einem Auslösen der Scharniere 130 bzw. der flexiblen Ausformungen zu einem Zuschnappen der Kiefer 110, 120 mit vorbestimmter Klemmkraft führt.

Im Einzelnen ist jeder Clip 100 einstückig aus einem Federblech gestanzt oder gelasert, indem aus dem Federblech ein Ring mit partiell unterschiedlicher

Ringbreite herausgearbeitet wird. Zwei diametrisch gegenüberliegende Ringabschnitte mit großer Ringbreite bilden die beiden Kiefer 110, 120, wohingegen die beiden dazwischen liegenden Ringabschnitte mit schmaler Ringbreite die Scharniere 130 bzw. die flexiblen (elastischen) Ausformungen ergeben. Die Kiefer 110, 120 sind dadurch ausgebildet, indem die Ringabschnitte mit großer Ringbreite über deren Flachseite bogenförmig gewölbt werden, wohingegen die beiden Ringabschnitte mit schmaler Ringbreite um ihre Längsachse um ca. 180° verdreht (tordiert) ausgebildet sind, um die Scharniere auszuformen. Durch diese besondere Formgebung des gelaserten Federblechs entsteht die Form einer Art Haifischmaul mit zwei aufeinander sich zu bewegenden Zahnreihen, welche durch Lasern der Ringabschnitte mit großer Ringbreite gebildet werden.

Die Funktionsweise des vorstehend beschriebenen medizinischen Gewebeclip 100 lässt sich wie Folgt beschreiben:

Im Allgemeinen stellt eine endoskopische Implantation einer medizinischen Vorrichtung insgesamt das für den Patienten verträglichste Verfahren dar. In diesem Fall muss die medizinische Vorrichtung von der Innenseite eines Hohlorgans an diesem fixiert werden. Zu diesem Zweck werden eine Anzahl (mindestens eine) der vorstehend beschriebenen Gewebeklammern, Clips oder Anker mittels eines Endoskops oder eines ähnlichen schaftartigen Zuführmittels in das Hohlorgan eingeführt und an vorbestimmten Stellen an der Organinnenseite platziert. Hiefür wird der jeweilige Clip oder Anker an das Organgewebe herangeführt und die Vorspannfeder für ein Zuschnappen des Clip oder Aufspannen des Ankers ausgelöst. Dieser hält oder klemmt daraufhin eine Gewebefalte zwischen seinen Kiefern oder seinen Haken oder Nadeln mit einer vorbestimmten Klemm- oder Spreizkraft ein, wobei sich die Zähne, Haken, Nadeln oder Zacken jedes Kiefers in das Gewebe bohren und dieses vorzugsweise durchdringen.

Das in der Fig. 1 nicht weiter dargestellte Endoskop oder schaftartige Zuführmittel ist in der Regel mit einem Endoskopkopf oder einer Endoskopkappe ausgerüstet, welche neben den für ein Endoskop generell erforderlichen Funktionen wie Beleuchtung, Optik und ggf. Spülvorrichtung zusätzlich eine Halte- und Abzieheinrichtung für den Gewebeclip aufweist. An dieser Stelle sei darauf hingewiesen, dass in dieser gesamten Anmeldung unter einem Endoskop auch eine einfache Einführhilfe ohne eigene Beleuchtung und Optik sowie Spülfunktion verstanden werden kann.

Die Halte- und Abzieheinrichtung besteht im Wesentlichen aus einer Spreizhülse sowie einem manuell oder ferngesteuert betätigbaren Schieber, der in Endoskoplängsrichtung bewegbar ist. Die Spreizhülse ist dabei derart ausgebildet, dass der bereits geöffnete Gewebeclip auf die Hülse aufgesetzt werden kann, derart, dass ein nach hinten Rutschen des Clip während dessen Einführung in das Hohlorgan verhinderbar ist. Zu diesem Zweck ist der Schieber axial hinter dem Clip positioniert und dient quasi als Axialanschlag für den Clip.

Sobald der Clip an einer bestimmten Stelle platziert werden soll, wird der Schieber axial nach vorne bewegt und streift dabei den Clip über die Spreizhülse ab. Dabei wird der Clip ausgelöst, d.h. der vorstehend anhand der Fig. 1 beschriebene Vorspannmechanismus innerhalb des Clip wird beim Abstreifen von der Spreizhülse freigegeben und die beiden Kiefer des Gewebeclip schnappen unter Einklemmen des dazwischen liegenden Gewebes zu. Es hat sich jedoch gezeigt, dass das genaue Setzen des Gewebeclip mit der bekannten Vorrichtung insbesondere im nahen Bereich des Schließmuskels sehr schwierig ist.

Angesichts dieses Stands der Technik besteht die Aufgabe der vorliegenden Erfindung daher darin, ein medizinisches, vorzugsweise proktologisches Instrument zum Setzen von Gewebeklammern zu schaffen, mittels dem auf einfache und sichere Weise ein Gewebeclip oder eine Gewebeklammer im Nahbereich des Schließmuskels in der inneren Organwand (Kolonwand) gesetzt werden kann

Diese Aufgabe wird durch medizinisches, vorzugsweise proktologisches Instrument gelöst, welche die technischen Merkmale gemäß dem Patentanspruch 1 aufweist. Vorteilhafte Ausgestaltungen der Erfindung sind dabei Gegenstand der Unteransprüche.

Erfindungsgemäß hat das proktologische Instrument einen Instrumentengriff, an welchem ein biegesteifer Rohrschaft an seinem proximalen Ende montiert ist, an dessen distalem Ende eine Kappe fixiert oder ausgebildet ist, auf die ein Gewebeclip vorzugsweise federelastisch aufgesetzt ist, der mittels einer Auslöse- oder Abziehvorrichtung von der Kappe abziehbar ist. Entweder die Kappe selbst oder der Rohrschaft ist in seinem distalen Endabschnitt in einem Bereich unmittelbar vor der Kappe in einem vorbestimmten fixen Winkel (> 0°) abgekröpft ist, sodass die von der Kappe definierte Abziehrichtung für den Clip in dem Winkel zur Rohrschaftsachse ausgerichtet ist. Auf diese Weise kann die Kappe besser an der Oberfläche der Organwand angesetzt und somit der Clip exakter positioniert werden.

Vorzugsweise ist die Auslöse- oder Abziehvorrichtung mit einem Zug- oder Druckelement ausgebildet, das längs des Rohrschafts in zumindest einem am Rohrschaft fixierten äußeren Funktionskanal verläuft und mit einem Betätigungshebel am Instrumentengriff gekoppelt ist. In vorteilhafter Weise ist das Zug- oder Druckelement an seinem distalen Ende mit einem Abziehring verbunden, der längsverschieblich auf der Kappe sitzt und mittels dem der Clip abziehbar ist. Dadurch kann ein Verkanten des Clip beim Abziehvorgang vermieden werden.

Weiter vorteilhaft ist es, den Rohrschaft in seinem distalen Endbereich unmittelbar vor der Kappe in S-Form auszubilden, wobei die Abziehrichtung des Kappe in dem fixen Winkel zur gedachten geraden Mittelachse des Rohrschafts verläuft. Durch die S-Form wird die Kappe parallel zur gedachten Mittelachse zurückversetzt (d.h. sie ragt weniger seitlich vor) und kann so besser in das Hohlorgan eingeführt werden.

Die Erfindung wird nachstehend anhand bevorzugter Ausführungsbeispiele unter Bezugnahme auf die begleitenden Figuren näher erläutert.

Es zeigen
Fig. 1 die beispielhafte Konstruktion eines Gewebeclips, wie er bereits aus dem Stand der Technik bekannt ist und wie er bei der vorliegenden Erfindung ebenfalls verwendet werden kann,
Fig. 2 eine Seitenansicht eines medizinischen Instruments gemäß einem ersten bevorzugten Ausführungsbeispiel der Erfindung,
Fig. 2a eine Vergrößerung des distalen Endabschnitts des medizinischen Instruments gemäß der Fig. 2,
Fig. 3 eine Perspektivendarstellung eines medizinischen Instruments gemäß einem zweiten bevorzugten Ausführungsbeispiel der Erfindung,
Fig. 4 eine Seitenansicht des medizinischen Instruments gemäß der Fig. 3 mit aufgezogenem Gewebeclip und abgeworfenen Gewebeclip,
Fig. 5 eine Seitenansicht eines medizinischen Instruments gemäß einem dritten bevorzugten Ausführungsbeispiel der Erfindung,
Fig. 6 eine Unteransicht eines medizinischen Instruments gemäß einem vierten bevorzugten Ausführungsbeispiel der Erfindung.
Fig. 7 eine Perspektivenansicht eines Gewebeclip gemäß einem bevorzugen Ausführungsbeispiel der Erfindung, welcher besonders zum Behandeln von Analfisteln entwickelt ist und
Fig. 8 eine Draufsicht einer alternativen Ausgestaltung des Gewebeclip gemäß der Fig. 7.

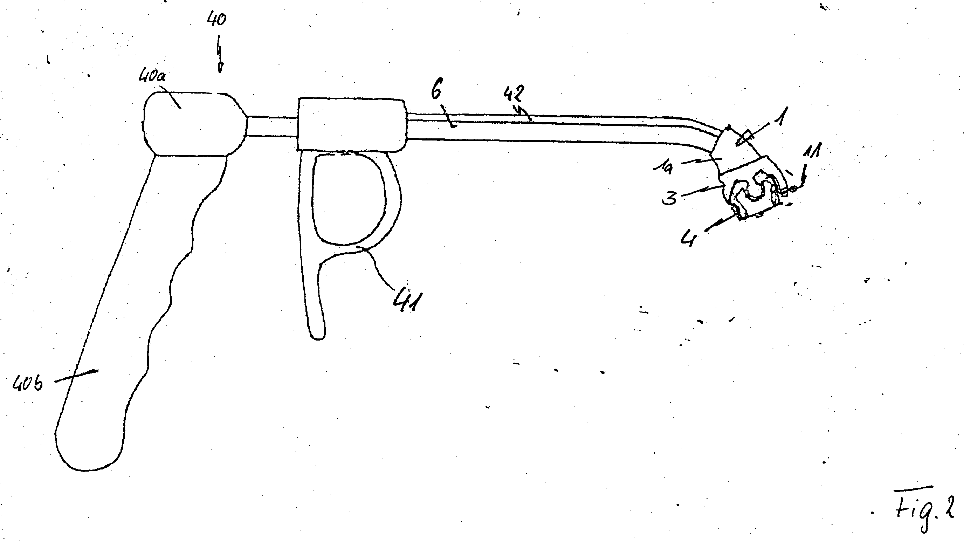

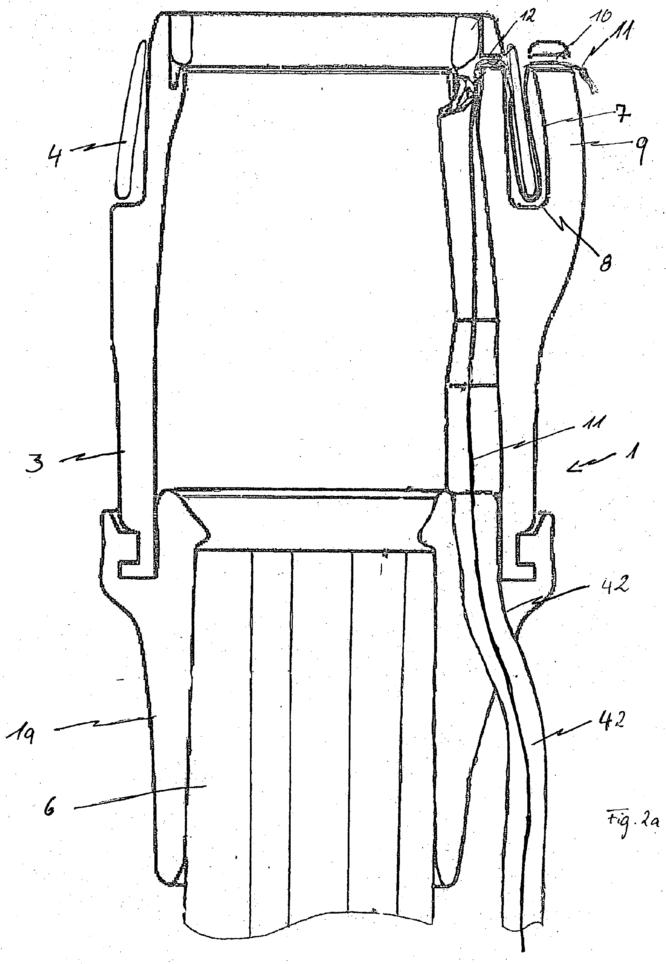

Gemäß der Fig. 2 und 2a besteht das medizinische (proktologische) Instrument aus einem vorzugsweise starren oder biegesteifen Rohrschaft 6 (beispielsweise aus einem nichtrostenden Stahl), an dessen proximalem Ende ein Betätigungsgriff 40 und an dessen distalem Ende ein Schaftkopf 1 mit einer Auslöse- oder Abziehvorrichtung für einen darauf aufgezogenen Gewebeclip 4 montiert ist.

Der Griff 40, vorzugsweise aus einem Kunststoffgußteil gefertigt, hat einen Montageabschnitt 40a, in den der Rohrschaft 6 eingesetzt, eingepresst, geklemmt oder eingegossen ist und einen Halteabschnitt 40b, der sich unter einem Winkel zum Rohrschaft 6 erstreckt. Des Weiteren ist ein Betätigungshebel 41 vorgesehen, der entweder längs des Rohrschafts 6 verschieblich auf diesem geführt ist oder der an dem Halteabschnitt 40b des Griffs 40 anscharniert ist. Der verschieblich geführte Betätigungshebel 41 ist direkt und der anscharnierte Betätigungshebel 41 ist über einen Getriebezug oder dergleichen Bewegungsübertragungs- oder Umlenkmechanismus (nicht dargestellt) mit einem Zug- und/oder Druckelement 11 gekoppelt, das vorliegend längs des Rohrschafts 6 in einer am Rohrschaft 6 fixierten Führung 42 beispielsweise in Form eines Bowdenzugs gelagert ist. Das Zug- und/oder Druckelement 11 ist wiederum mit einer Gewebeclipabwurfeinrichtung gekoppelt, mittels der ein auf den Schaftkopf 1 vorzugsweise federelastisch aufgezogener Gewebeclip 4 in der vorstehend anhand der Fig. 1 beschriebenen Bauform aufgezogen ist.

Im Konkreten besteht der Schaftkopf 1 gemäß der Fig. 2a aus einer Art Kappe (aus einem Kunststoffmaterial), die einen Aufsteckabschnitt 1 a (vorzugsweise eine Silikontülle) hat, der im montierten Zustand den distalen Endabschnitt des starren Rohrschafts 6 umgibt.

Die Kappe 1 ist im Axialabstand zum Aufsteckabschnitt 1a mantelseitig zu oder mit einer Spreizhülse (Spreizhülsenabschnitt) 3 ausgeformt, die im vorliegenden Ausführungsbeispiel mit dem Aufsteckabschnitt 1 a formschlüssig verbunden (verclipt) ist. Sie kann aber auch einstückig mit dem Aufsteckabschnitt 1a verbunden oder an diesem angeklebt oder angeschweißt sein. Auf die Spreizhülse 3 ist der Gewebeclip 4 aufschiebbar, wie er vorstehend, anhand der Fig. 1 näher beschrieben wurde und somit ebenfalls zum Erfindungsgegenstand zählt. Die Spreizhülse 3 steht über die distale Stirnseite des Rohrschafts 6 axial vor und bildet somit einen an ihrer vorderen Kante radial nach Außen abgerundeten becher- oder napfförmigen Hülsenabschnitt.

Vorzugsweise ist der Aufsteckabschnitt 1 a auf das distale Endstück des Rohrschafts 6 reibschlüssig aufgesteckt. Es kann aber auch aufgeklebt, mit dem Rohrschaft 6 verpresst oder vergossen sein.

Die Spreizhülse 3 gemäß der Erfindung weist eine von ihrer distalen Stirnseite aus in Axialrichtung eingebrachte Stirnnut 7 in der mantelseitigen Kappen- bzw. Hülsenwand auf, welche sich vorzugsweise als teilkreis- bzw. sichelförmiger (Umfangs-) Schlitz an der distalen Stirnseite der Spreizhülse 3 öffnet und deren Nutengrund an einer axial hinteren Stelle, vorzugsweise etwa in einem axial mittleren Abschnitt der Spreizhülse 3 einen Anschlag 8 bildet. Der Radius der Stirnnut 7 ist jedoch größer gewählt als der Außenradius der Spreizhülse 3, sodass die Hülsenwand bei der Ausbildung der Stirnnut 7 zwei in Umfangsrichtung entsprechend beabstandete Schlitze erhält. Durch Ausbilden dieser Stirnnutschlitze wird die Kappenmantelwand in diesem Bereich somit längsgespalten, wodurch an der Außenseite der Kappenwand eine Art Lasche oder Zunge 9 entsteht, welche die radial äußere Nutenwand definiert.

Eine weitere Variante der Bereitstellung einer Stirnnut gemäß vorstehender Definition ist die zusätzliche Anordnung einer vorzugsweise in Axialrichtung gekrümmten Lasche oder Zunge, wie dies insbesondere in der Fig. 2a gezeigt ist, deren Wurzel einstückig mit der Kappe ausgebildet ist und die sich unter Ausbildung der Nut im Radialabstand zur Kappenmantelwand axial in Richtung der Spreizhülse erstreckt. In diesem Fall wird also die Mantelwand nicht gespalten (wie vorstehend beschrieben), sondern es wird ein zusätzliches Bauteil in Form der Lasche über die Mantelwand der Kappe geführt. Diese Lasche kann derart schmal dimensioniert sein, dass sie im Querschnitt geradlinig (ohne Radius) bleibt, d.h. sie muss nicht notwendiger Weise dem Kappenumfang folgen. Darüber hinaus kann die Grundrissform der Lasche dann weitestgehend beliebig gestaltet werden, d.h. sie kann sich in Richtung Laschenwurzel (Übergangsbereich zwischen Lasche und Kappe) verdicken und/oder verbreitern, um so eine größere Steifigkeit zu erhalten. Auch die Laschenwurzel selbst kann nach statischen Gesichtspunkten zur Erreichung einer möglichst hohen Steifigkeit frei dimensioniert und gestaltet werden.

Unabhängig davon, nach welcher Fertigungsvariante die Lasche 9 letztlich gebildet wird, erstreckt sie sich erfindungsgemäß vom, den Anschlag 8 darstellenden Nutengrund in Richtung zur distalen Stirnseite der Kappe 1 bzw. der Spreizhülse 3, wobei deren abgerundete freie Vorderkante gegenüber der distalen Vorderkante der Spreizhülse 3 axial geringfügig zurückgesetzt ist.

Wie in der Fig. 2a zumindest angedeutet ist, erstreckt sich die Stirnnut 7 nicht exakt parallel zur Kappenmittelachse sondern ist in Richtung distaler Stirnseite zur Mittelachse hin geneigt, sodass ein darin eingesteckter Clip 4 leichter nach vorn abgleiten kann. Darüber hinaus ist die Nut 7 nicht geradlinig sondern deren Nutenwände, zumindest die äußere Nutenwand, sind in Axialrichtung leicht gekrümmt, derart, dass sich die Nut 7, zumindest die Lasche 9 in ihrem axialen Mittenabschnitt radial nach Außen wölbt. Auf diese Weise wird das Zusammenklappverhalten eines abgleitenden Gewebeclip 4 mit der Konstruktion gemäß der Fig. 1 bereits in diesem Zustand geometrisch zugelassen bzw. erleichtert.

In einem axial vorderen Endabschnitt der Lasche 9 ist diese mit einer radialen äußeren Durchgangsbohrung 10 versehen, durch die ein Faden 11, Kabel oder Gewebe vom Nuteninneren in Richtung nach Außen der Kappe 1 geführt und darin fixiert ist. Der Faden 11, Kabel oder Gewebe bildet dabei das vorstehend genannte Zugelement. Vorzugsweise ist das eine Fadenende zu diesem Zweck an der Laschenaußenseite verknotet, sodass ein Zurückziehen des Fadens 11 durch die radiale Durchgangsbohrung 10 verhindert wird. Des Weiteren ist die Kappe 1 an einer Stelle im Wesentlichen radial gegenüberliegend zur vorstehend genannten Durchgangsbohrung 10, d.h. im distalen Endbereich der axial überstehenden Spreizhülse 3 mit einer radialen inneren Durchgangsbohrung 12 versehen, durch welche der Faden 11 vom Nuteninneren nach Innen in die Spreizhülse 3 geführt ist. Der die Nut durchquerende Fadenabschnitt bildet dabei die genannte Auslöse- oder Abziehvorrichtung für den Clip.

Wie insbesondere aus der Fig. 2a zu entnehmen ist, befindet sich die innere Durchgangsbohrung 12 axial vor der distalen Stirnseite des Rohrschafts 6, sodass der Faden 11 aus der inneren Durchgangsbohrung 12 kommend in einen an der Schaft-Stirnseite sich öffnenden, an der Außenseite des Rohrschafts 6 verlaufenden Funktionskanal (Führung) 42 einfädelbar ist, welcher die vorstehend genannte Führung des Zugelements 11 bildet.

Der Rohrschaft 6 ist des Weiteren im Bereich seines distalen Endabschnitts (d.h. in einem Bereich unmittelbar vor der Kappe 1) abgekröpft oder gebogen, sodass die durch die Spreizhülse 3 definierte Abwurfrichtung für den Gewebeclip 4 in einem (fixen) Winkel (> 0°) zur Rohrachse verläuft.

Die Arbeitsweise des erfindungsgemäßen medizinischen Instruments mit der Halte- und Abziehfunktion für den Gewebeclip 4 wird nachfolgend näher beschrieben.

Um einen Gewebeclip 4 beispielsweise gemäß der Fig. 1 an seine vorbestimmte Position zu bringen, muss dieser zuerst auf die Spreizhülse 3 der Schaftkappe 1 aufgezogen werden. Hierfür werden Unter- und Oberkiefer des Gewebeclips 4 von Hand aufgeklappt, sodass der Clip 4 an der gerundeten Vorderkante der Spreizhülse 3 angesetzt und über diese geschoben werden kann. Dabei dringt die Hinterkante des Gewebeclips 4 in die Stirnnut 7 der Schaftkappe 1 ein und zieht dabei den Faden 11 aus dem Funktionskanal 42 am Instrumentenschaft 6 heraus.

Schließlich kommt die Schiebebewegung des Clips 4 mit dessen in Anlage kommen am Nutengrund 8 zum Stillstand, wobei der Clip 4 sowie der mitgenommene Faden 11 die in Fig. 2 gezeigte Raumlage einnehmen. D.h. in dieser Lage ist der Clip 4 vollständig auf die Kappe 1 aufgezogen und kann so über den Rohrschaft 6 in ein Hohlorgan eingebracht werden. Der Faden 11 umgreift dabei die Hinterkante des Clip 4 und erhält somit eine U-Form in Fadenlängsrichtung gesehen.

Sobald das erfindungsgemäße proktologische Instrument an eine erkrankte Stelle innerhalb eines Hohlorgans gelangt ist, wird die Spreizhülse 3 gegen die Organwand gedrückt. Soll der Clip 4 nunmehr abgestreift werden, wird der Faden 11, welcher durch den Funktionskanal 42 bis zum proximalen Betätigungshebel 41 geführt ist, durch Längsverschieben des Betätigungshebels 41 gezogen, wobei sich der die Stirnnut 7 in Radialrichtung durchquerende Fadenabschnitt verkürzt. Da der Faden 11 in der äußeren Durchgangsbohrung 10 fixiert ist, übt dieser mit einer entsprechenden Übersetzung nach dem Flaschenzugprinzip auf den Clip 4 eine Kraft in Axialrichtung aus, wodurch der Clip 4 in Richtung distales Ende der Endoskopkappe 1 verschoben wird. Durch die äußere Abrundung der vorderen Spreizhülsenkante sowie die weiche, d.h. gewölbte Formgebung der Stirnnut 7 (insbesondere der Lasche 9) wird ein Abgleiten des Clip 4 über die Vorderkante der Spreizhülse 3 erleichtert und die über den Faden 11 aufzubringende maximale Verschiebekraft weiter reduziert. Sobald die Hinterkante des Clips 4 aus der Stirnnut 7 getreten ist und daher nicht mehr von der Lasche 9 gehalten werden kann, bewirkt die im Clip 4 gespeicherte Vorspannkraft ein Abspringen des Clips 4 von der Spreizhülse 3, wodurch die Organwand im Bereich unmittelbar vor der Spreizhülse 3 abgeklemmt wird.

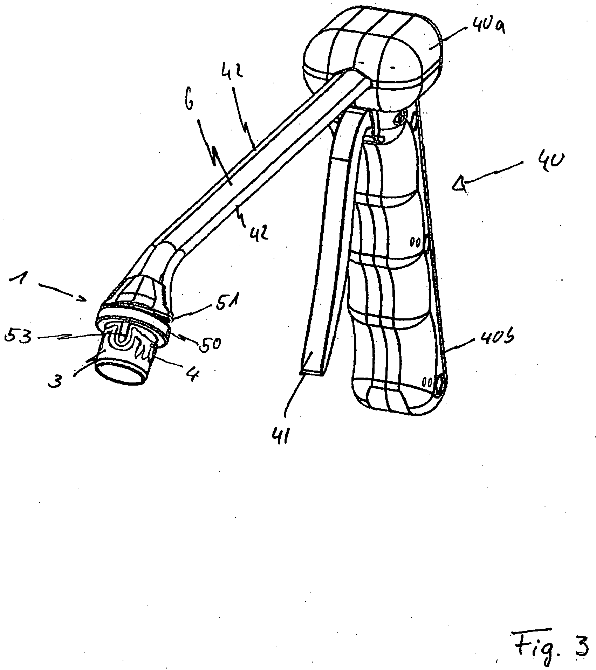

An dieser Stelle sei darauf hingewiesen, dass an Stelle des verschieblich gelagerten Betätigungshebels 41 gemäß der Fig. 2 auch ein am Halteabschnitt des Griffs 2 anscharnierter Hebel 41 gemäß der Fig. 3 vorgesehen sein kann, an dem der Faden 11 so angelenkt ist, dass dieser bei einem Verschwenken des Hebels 41 zum Halteabschnitt 40b hin gezogen wird.

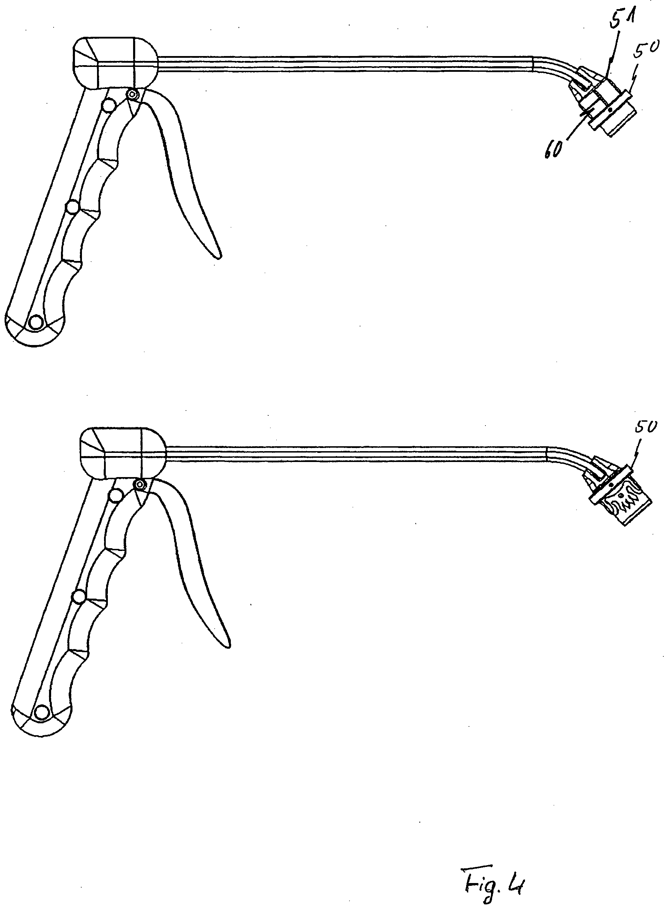

Die Fig. 3 und 4 zeigt dabei ein weiteres Ausführungsbeispiel der Erfindung, wobei im Nachfolgenden lediglich auf die zu dem vorstehenden Ausführungsbeispiel unterschiedlichen Merkmale eingegangen werden soll.

Wie aus der Fig. 3 und 4 zu entnehmen ist, besteht die Abziehvorrichtung des zweiten bevorzugten Ausführungsbeispiels der Erfindung aus einem Abziehring 50, der auf die Spreizhülse 3 gezogen ist und sich gegen einen äußeren Wellenabsatz 51 im Mittenbereich der Schaftkappe 1 anlegt. Alternativ hierzu kann sich der Abziehring 50 aber auch gegen den Nutengrund einer Stirnnut gemäß dem vorstehenden Ausführungsbeispiel axial anlegen.

Im zweiten Ausführungsbeispiel ist jedoch keine Lasche oder Stirnnut vorgesehen. Statt dessen ist der durch die vorstehend beschriebene innere Durchgangsbohrung durchgeführte Faden unmittelbar am Abziehring 50 befestigt, indem in diesen eine Durchgangsbohrung (in Längsrichtung) eingebracht ist, durch die der Faden geführt und darin fixiert ist. Des Weiteren ist am Außenumfang der Spreizhülse 3 ein axialer Steg 53 oder eine Axialnut ausgeformt, der in eine axiale Innennut des Abziehrings 50 oder ein axialer Innensteg greift und eine Axialführung für den Abziehring 50 bildet. Im Übrigen ist der Abziehring 50 hinsichtlich seiner Form dem Gewebeclip vorzugsweise angeglichen, sodass dieser im Wesentlichen passgenau an den Ring 50 angelegt werden kann und damit eine vorbestimmte (Dreh-) Position auf der Spreizhülse 3 einnimmt. Es können natürlich auch zwei Fäden in zwei Funktionskanälen 42 vorgesehen sein.

Die Funktionsweise des medizinischen Instruments des zweiten Ausführungsbeispiels der Erfindung lässt sich anhand der Fig. 4 wie Folgt beschreiben.

Sobald die Spreizhülse 3 auf eine erkrankte Stelle der Organwand aufgesetzt ist (das flächige Aufsetzen der Spreizhülse 3 wird durch den abgekröpften Rohrschaft 6 erleichtert), wird der Gewebeclip 4 abgezogen. Hierzu muss der Faden 11 längs des Rohrschafts 6 gezogen werden, wodurch sich der Abziehring 50 nach vorn in Richtung zur distalen Stirnkante der Spreizhülse 3 bewegt. Dabei wird auch der Clip 4 nach vorn verschoben, bis dieser durch dessen Federvorspannung über die distale Stirnkante der Spreizhülse 3 abspringt und das Organgewebe zwischen seinen Kiefern einklemmt.

An dieser Stelle sei auf eine zum vorstehend beschriebenen Faden alternative Ausführungsform eines Zug- und/oder Druckelements hingewiesen.

Bisher wurde die Auslöse- oder Abziehvorrichtung für den Gewebeclip 4 durch eine Zugbewegung des Fadens 11 betätigt. Es kann aber auch so sein, dass die Betätigung des Betätigungshebels 41 zu einer Druckbewegung des Druckelements führt, wodurch die Auslöse- oder Abziehvorrichtung nicht nach vorn gezogen sondern geschoben wird. Diese Alternative ist insbesondere in der Fig. 4 angedeutet. In diesem Fall ist beispielsweise ein Draht oder eine biegsame Schubstange 60 in vorzugsweise zwei seitlich am Rohrschaft 6 angeordneten Führungskanälen 42 jeweils gelagert, die jeweils an ihrem distalen Ende mit dem Abziehring 50 verbunden sind. Wird der anscharnierte oder verschieblich gelagerte Betätigungshebel 41 verschwenkt/verschoben, wird diese Bewegung von den vorzugsweise zwei Schubstangen 60 auf den Abziehring 50 übertragen und dadurch der Clip 4 ausgelöst.

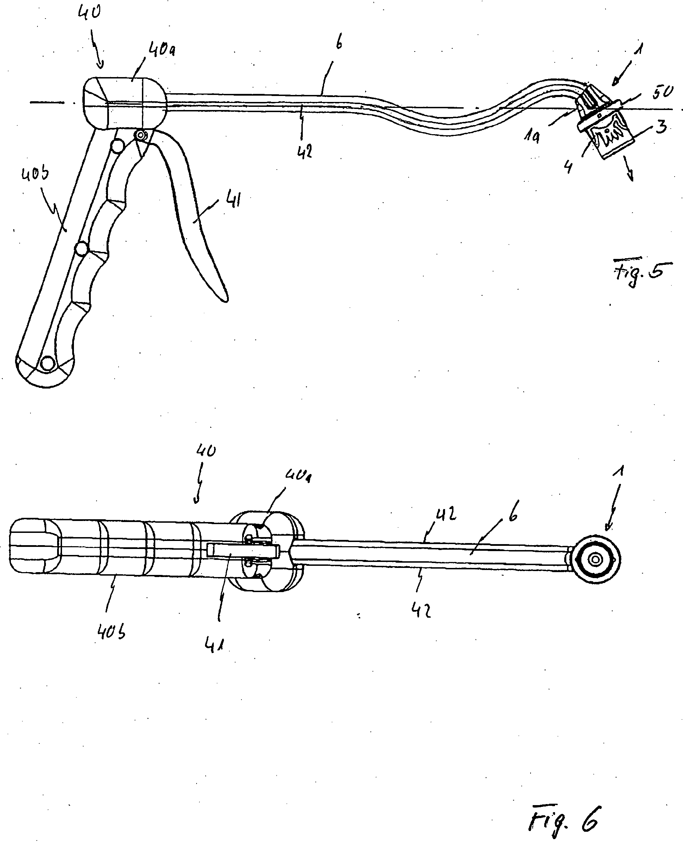

Schließlich ist in der Fig. 5 ein drittes bevorzugtes Ausführungsbeispiel der Erfindung gezeigt, welches auf dem zweiten Ausführungsbeispiel basiert. In sofern werden nachfolgend nur die zum zweiten Ausführungsbeispiel unterschiedlichen Merkmale beschrieben.

Gemäß der Fig. 5 ist der starre Rohrschaft 6 nicht einfach am distalen Endabschnitt abgekröpft, sondern weist in diesem Bereich eine Schwanenhals- bzw. S-Form auf, wobei die durch die Spreizhülse 3 definierte Abwurfrichtung in einem Winkel (> 0°) bezüglich des geraden Rohrschaftabschnitts im Mittelbereich und proximalen Bereich des Rohrschafts 6 ausgerichtet ist.

Durch diese Formgebung kann die Spreizhülse 3 bezüglich einer (gedachten) Mittellinie des Rohrschafts 6 parallel hierzu zurückversetzt werden, sodass ein in den Kolon eines Patienten eingeschobenes Instrument die Organwandung nicht oder nur geringfügig aufweitet.

Des Weiteren kann, wie in der Fig. 6 angedeutet ist, der Halteabschnitt 40b des Instrumentengriffs 40 bezüglich des Rohrschafts 6 umgeklappt werden. Hierfür ist der Halteabschnitt 40b über ein Scharnier am Montageabschnitt 40a des Griffs 40 angelenkt und ggf. dort verrastet. Durch Umklappen des Haltegriffs 40b ergibt sich eine Schraubenzieher-Griffposition, wodurch die Handhabbarkeit des Instruments in eingeführter Position verbessert wird.

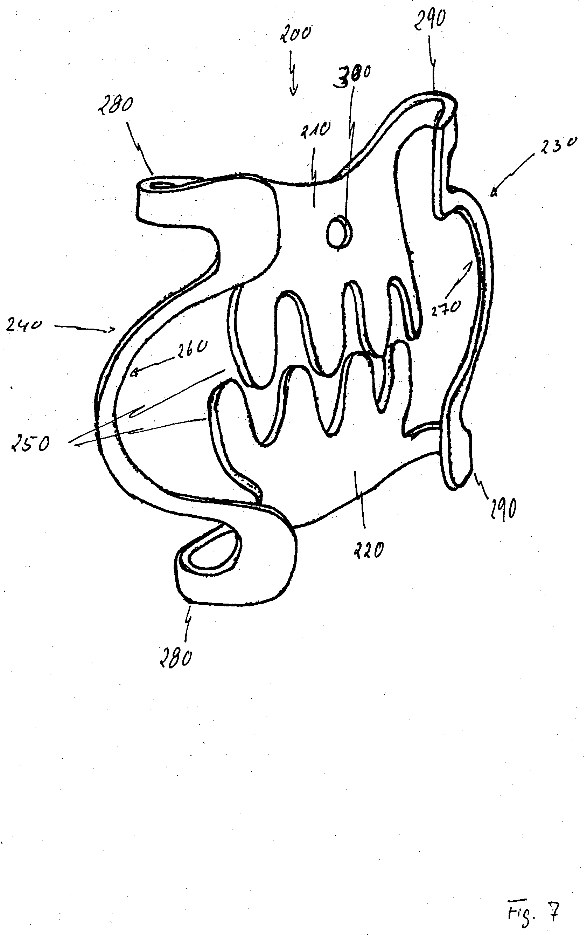

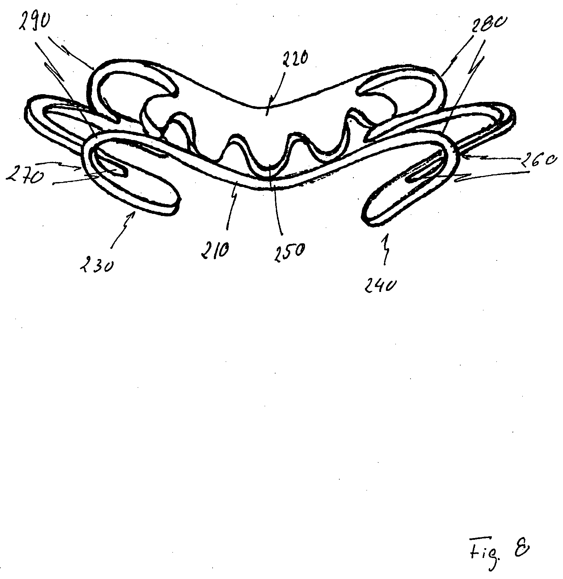

In den Fig. 7 und 8 ist ein Gewebeclip dargestellt, der besonders für das erfindungsgemäße proktologische Instrument entwickelt wurde.

Dieser Clip 200 besteht ebenfalls aus einer maulartigen Klemmeinrichtung, die einen gezahnten Oberkiefer 210 und einen gezahnten Unterkiefer 220 hat. Im Konkreten ist der erfindungsgemäße Clip 200 aus einem Blatt- oder Blechmaterial einstückig ausgestanzt oder gelasert. Er besteht im Wesentlichen aus einem geschlossenen Ring mit den zwei diametral sich gegenüberliegenden Ober- und Unterkieferabschnitten 210, 220, die jeweils aus einem breiten Blattstück gebildet sind und zwei diametral gegenüberliegenden, sowie jeweils 90° zu den Kieferabschnitten versetzt angeordneten Scharnierabschnitten 230, 240, welche die Kieferabschnitte 210, 220 miteinander verbinden.

Die Kieferabschnitte 210, 220 sind an ihren jeweils zugewandten Längskanten mit Zacken oder Zähnen 250 ausgeformt, die den Ring durchqueren und ineinandergreifen. Die Scharnierabschnitte 230, 240 sind aus einem gegenüber den Kieferabschnitten 210, 220 wesentlich schmaleren leistenförmigen Blattstück jeweils gebildet, das über dessen jeweils gesamten Kreissegmentumfang im wesentlichen halbkreisförmig nach Innen, das heißt in Richtung zum Ringmittelpunkt ausgewölbt ist. Hierdurch entstehen am Ring zwei diametral sich gegenüberliegende, halb- oder teilkreisförmige Ausbuchtungen 260, 270, welche die Scharniere der beiden Kieferabschnitte 210, 220 bilden.

Des Weiteren ist der der Ring über seine Flachseite im Bereich der beiden Scharnierabschnitte um ca. 180° jeweils umgebogen oder gefaltet. Im Konkreten sind die beiden Scharnierabschnitte 230, 240 senkrecht zur Ringebene in ihren jeweiligen Übergangsbereichen 280, 290 zum Ober- und Unterkiefer derart umgebogen, dass sich die halb- oder teilkreisförmige Ausbuchtung 260, 270 nach Außen, d.h. weg vom Ringmittelpunkt ausrichtet. Darüber hinaus sind de Kieferabschnitte 210, 220 über deren gesamten Kreisumfangsabschnitt ebenfalls senkrecht zur Ringebene gebogen oder gewölbt, jedoch in die zur Umbiegrichtung der Scharnierabschnitte 230, 240 entgegen gesetzte Richtung.

Durch diese Wölbung ergibt sich ein dreidimensionaler Ring, bei dem die Scharniereabschnitte 230, 240 oberhalb (auf der Wölbungsaußenseite) der Kieferabschnitte 210, 220 zu liegen kommen und sich radial nach Außen sowie schräg in Richtung Kieferabschnitte 210, 220 ausrichten. An dieser Stelle sei noch darauf hingewiesen, dass das Clipmaterial zumindest im Bereich der Scharnierabschnitte 230, 240 und vorzugsweise über den gesamten Clipring hoch elastisch ist.

Wird der in der Fig. 7 in Konstruktionslage (entspannt) dargestellte Clip 200 elastisch aufgeklappt, das heißt werden die Kieferabschnitte 210, 220 geöffnet, erfahren die beiden Scharnierabschnitte 230, 240 eine elastische Verformung, dergestalt, dass sich die halb- oder teilkreisförmigen Auswölbungen 260, 270 einengen. Gleichzeitig werden die Scharnierabschnitte 230, 240 tordiert, derart, dass die Auswölbungen 260, 270 in Richtung 90°-Winkel bezüglich der Kieferabschnittsflächen drehen. Durch beide elastische Bewegungen wird eine Federenergie gespeichert, welche die beiden Kieferabschnitte 210, 220 mit einer vorbestimmten Schließkraft vorspannt.

Abschließend sei noch die in der Fig. 7 gezeigte Durchgangsbohrung 300 in einem der Kieferabschnitte 210 erwähnt, in die ein Faden oder dergleichen Befestigungselement (nicht gezeigt) einfädelbar ist. In diesem Fall kann der Gewebeclip 200 als Anker für ein medizinisches Instrument oder dergleichen verwendet werden.

In der Fig. 8 ist eine alternative Ausgestaltung des Gewebeclip 200 gemäß der Fig. 7 dargestellt, wobei nachfolgen lediglich die geometrischen Unterschiede zum Clip 200 gemäß der Fig. 7 beschrieben werden sollen.

Beim Clip gemäß der Fig. 8 sind die halb- oder teilkreisförmigen Auswölbungen 260, 270 der Scharnierabschnitte 230, 240 in ungebogenem Zustand nach Außen, d.h. weg vom Ringmittelpunkt ausgerichtet und bilden somit zwei diametral gegenüberliegende Ausbeulungen am Ringumfang. Werden die Scharnierabschnitte 230, 240 nunmehr im vorstehenden Sinne umgebogen, werden hierdurch die ursprünglich nach Außen gerichteten Ausbeulungen nunmehr nach Innen gerichtet. Im fertigen Zustand des Clip 200 sind demzufolge die Auswölbungen 260, 270 der Scharnierabschnitte 230, 240 gemäß der Fig. 8 exakt 180° entgegen gesetzt zu den Auswölbungen der Scharnierabschnitte gemäß der Fig. 7 ausgerichtet. Alle übrigen geometrischen Merkmale sind identisch und auch die Funktionsweise des Gewebeclip 200 gemäß der Fig. 8 entspricht jener der Fig. 7, sodass an dieser Stelle auf die vorstehende Beschreibung verwiesen werden kann.

## Patentansprüche

1. Proktologisches Instrument mit einem Instrumentengriff (40), an welchem ein biegesteifer Rohrschaft (6) an seinem proximalen Ende montiert ist, an dessen distalem Ende eine Kappe (1) fixiert oder ausgebildet ist, auf die ein Gewebeclip (4, 200) vorzugsweise federelastisch aufgesetzt ist, der mittels einer Auslöse- oder Abziehvorrichtung von der Kappe (1) abziehbar ist, **dadurch gekennzeichnet, dass** der Rohrschaft (6) in seinem distalen Endabschnitt in einem Bereich unmittelbar vor der Kappe (1) und/oder die Kappe (1) selbst in einem vorbestimmten fixen Winkel abgekröpft ist, sodass die von der Kappe (1) definierte Abziehrichtung für den Clip (4, 200) in dem Winkel zur Rohrschaftsachse ausgerichtet ist, wobei die Auslöse- oder Abziehvorrichtung zumindest ein Zug- und/oder Druckelement (11, 60) aufweist, das sich längs des Rohrschafts (6) in zumindest einem außerhalb am Rohrschaft (6) fixierten Funktionskanal (42) erstreckt und an seinem proximalen Ende an einen Betätigungshebel (41) des Instrumentengriffs (40) gekoppelt ist.

2. Proktologisches Instrument nach Anspruch 1, **dadurch gekennzeichnet, dass** der biegesteife Rohrschaft (6) im Bereich seines distalen Endabschnitts S-förmig gebogen ist.

3. Proktologisches Instrument nach einem der vorstehenden Ansprüche, **dadurch gekennzeichnet, dass** das zumindest eine Zug- und/oder Druckelement (11, 60) an seinem distalen Ende an einen Abziehring (50) gekoppelt ist, der axialverschieblich auf der Kappe (1) geführt ist.

4. Proktologisches Instrument nach einem der Ansprüche 1 bis 3, **dadurch gekennzeichnet, dass** zwei Funktionskanäle (42) diametral gegenüberliegend an der Außenseite des Rohrschafts (6) befestigt sind und die Zug- und/oder Druckelemente (11, 60) in Form zweier biegeflexibler Drähte oder Wellen ausgebildet sind, die in den Funktionskanälen axialverschieblich gelagert sind, um eine Zug- und/oder Druckkraft achssymmetrisch auf den Abziehring (50) aufzubringen.

5. Proktologisches Instrument nach einem der vorstehenden Ansprüche 1 und 2, **dadurch gekennzeichnet, dass** die Kappe (1) an ihrer Außenseite eine längs sich erstreckende Lasche (9) hat, die zwischen sich und der Kappenaußenwand einen nutenförmigen Spalt (7) ausbildet, in den der Clip (4, 200) eingeschoben ist.

6. Proktologisches Instrument nach Anspruch 5, **dadurch gekennzeichnet, dass** das Zug- und/oder Druckelement ein Faden (11) ist, der an seinem distalen Ende quer durch den nutenförmigen Spalt geführt und an der Lasche (9) fixiert ist, um bei Aufschieben des Clip (4) von diesem mitgezogen zu werden und der an seinem proximalen Ende an an einem Betätigungshebel (41) des Instrumentengriffs (40) befestigt ist, um bei Betätigen des Hebels (41) gezogen zu werden, wodurch sich der Fadenabschnitt innerhalb des Spalts (7) verkürzt und dabei den Clip (4, 200) nach vorn abzieht.

7. Proktologisches Instrument nach einem der vorstehenden Ansprüche, **dadurch gekennzeichnet, dass** der Instrumentengriff (40) einen Halteabschnitt (40b) hat, dessen Winkelstellung bezüglich des Rohrschafts (6) verstellbar ist.

## Claims

1. A proctological instrument comprising an instrument handle (40) to which at its proximal end a bending-resistant tubular shaft (6) is mounted, at the distal end of which a cap (1) is fixed or formed to which a tissue clip (4, 200) adapted to be withdrawn from the cap (1) by means of a releasing or withdrawing means is attached in a preferably spring-elastic manner, **characterized in that** the tubular shaft (6) is offset at its distal end portion, in an area directly ahead of the cap (1) and/or the cap (1) itself, at a predetermined fixed angle so that the withdrawing direction for the clip (4, 200) defined by the cap (1) is aligned at the angle with respect to the tubular shaft axis, wherein the releasing or withdrawing device includes at least one pull and/or push element (11, 60) extending along the tubular shaft (6) in a respective function channel (42) fixed at the outside of the tubular shaft (6) and being coupled at its proximal end to an operating lever (41) of the instrument handle (40).

2. The proctological instrument according to claim 1, **characterized in that** the bending-resistant tubular shaft (6) is bent in an S-shape in the area of its distal end portion.

3. The proctological instrument according to any one of the preceding claims, **characterized in that** at least one pull and/or push element (11, 60) is coupled at its distal end to a slip ring (50) which is guided so as to be axially movable on the cap (1).

4. The proctological instrument according to any one of the preceding claims 1 to 3, **characterized in that** two function channels (42) are mounted diametrally opposed to the outside of the tubular shaft (6) and the pull and/or push elements (11, 60) are in the form of two flexible wires or shafts that are supported to be axially movable in the function channels so as to apply a pulling and/or pushing force to the slip ring (50) in an axially symmetrical manner.

5. The proctological instrument according to any one of the preceding claims 1 and 2, **characterized in that** the cap (1) includes at its outside a longitudinally extending tab (9) forming a groove-shaped gap (7) between itself and the outer cap wall, wherein the clip (4, 200) is inserted into this gap.

6. The proctological instrument according to claim 5, **characterized in that** the pull and/or push element is a thread (11) which is guided at its distal end across the groove-shaped gap and is fixed at the tab (9) in order to be entrained by the same when the clip (4) is slipped on and which is mounted at its proximal end to an operating lever (41) of the instrument handle (40) in order to be pulled upon operation of the lever (41), whereby the thread portion inside the gap (7) shrinks and thus withdraws the clip (4, 200) to the front.

7. The proctological instrument according to any one of the preceding claims, **characterized in that** the instrument handle (40) includes a holding portion (40b) whose angular position is adjustable with respect to the tubular shaft (6).

## Revendications

1. Instrument proctologique comprenant un manche d'instrument (40), au niveau duquel une tige tubulaire (6) rigide en flexion est montée au niveau de son extrémité proximale, au niveau de l'extrémité distale de laquelle un capuchon (1) est fixé ou est réalisé, sur lequel est placé de préférence de manière élastique un clip tissulaire (4, 200), qui peut être extrait du capuchon (1) au moyen d'un dispositif de détachement ou d'extraction, **caractérisé en ce que** la tige tubulaire (6) dans son segment d'extrémité distal dans une zone directement devant le capuchon (1) et/ou le capuchon (1) lui-même sont coudés selon un angle fixe prédéterminé de sorte que la direction d'extraction, définie par le capuchon (1), pour le clip (4, 200) est orientée dans l'angle par rapport à l'axe de tige tubulaire, sachant que le dispositif de détachement ou d'extraction présente au moins un élément de traction et/ou de pression (11, 60), qui s'étend le long de la tige tubulaire (6) dans au moins un canal fonctionnel (42) fixé à l'extérieur au niveau de la tige tubulaire (6) et est couplé, au niveau de son extrémité proximale, au niveau d'un levier d'actionnement (41) du manche d'instrument (40).

2. Instrument proctologique selon la revendication 1, **caractérisé en ce que** la tige tubulaire (6) rigide en flexion est pliée de manière à présenter une forme de S dans la zone de son segment d'extrémité distal.

3. Instrument proctologique selon l'une quelconque des revendications précédentes, **caractérisé en ce que** l'élément de traction et/ou de pression (11, 60) au moins au nombre de un est couplé, au niveau de son extrémité distale, au niveau d'une bague d'extraction (50), qui est guidée sur le capuchon (1) de manière à pouvoir coulisser axialement.

4. Instrument proctologique selon l'une quelconque des revendications 1 à 3, **caractérisé en ce que** deux canaux fonctionnels (42) sont fixés de manière diamétralement opposée au niveau du côté extérieur de la tige tubulaire (6), et **en ce que** les éléments de traction et/ou de pression (11, 60) sont réalisés sous la forme de deux fils métalliques flexibles en flexion ou sous la forme d'arbres, qui sont montés de manière à pouvoir coulisser axialement dans les canaux fonctionnels afin d'appliquer une force de traction et/ou de pression avec une symétrie axiale sur la bague d'extraction (50).

5. Instrument proctologique selon l'une quelconque des revendications 1 et 2, **caractérisé en ce que** le capuchon (1) comprend, au niveau de son côté extérieur, une bride (9) s'étendant en longueur, qui réalise, entre elle et la paroi extérieure de capuchon, une fente (7) présentant une forme de rainure, dans laquelle le clip (4, 200) est enfilé.

6. Instrument proctologique selon la revendication 5, **caractérisé en ce que** l'élément de traction et/ou de pression est un fil (11), qui est guidé, au niveau de son extrémité distale, de manière transversale à travers la fente présentant une forme de rainure et est fixé au niveau de la bride (9) afin d'être entraîné par le clip (4) lors de son enfilement, et qui est fixé, au niveau de son extrémité proximale, au niveau d'un levier d'actionnement (41) du manche d'instrument (40), afin d'être tiré lors de l'actionnement du levier (41), ce qui permet de raccourcir le segment de fil à l'intérieur de la fente (7) et d'extraire vers l'avant le clip (4, 200).

7. Instrument proctologique selon l'une quelconque des revendications précédentes, **caractérisé en ce que** le manche d'instrument (40) comprend un segment de maintien (40b), dont la position angulaire peut être ajustée par rapport à la tige tubulaire (6).
